# EUROPEAN PATENT APPLICATION

(11) **EP 4 553 843 A1**
(43) Date of publication of application: **14.05.2025**
(21) Application number: 23208966.4
(22) Date of filing: 10.11.2023
(51) Int. Cl.: G16H 20/40, G16H 50/20, G06T 13/00, G06T 17/00

(54) **METHOD FOR PATIENT POSITIONING IN A MEDICAL PROCEDURE AND MEDICAL SYSTEM**

(71) Applicant: Koninklijke Philips N.V., 5656 AG Eindhoven (NL)
(72) Inventor: GOOßEN, Andre, Eindhoven (NL); VON BERG, Jens, Eindhoven (NL); BYSTROV, Daniel, 5656 AG Eindhoven (NL); PAUL, Soubhik, Eindhoven (NL); YOUNG, Stewart Matthew, Eindhoven (NL); BRUECK, Heiner Matthias, Eindhoven (NL); KROENKE-HILLE, Sven, Eindhoven (NL); HARDER, Tim Philipp, Eindhoven (NL)
(74) Representative: Philips Intellectual Property & Standards

(57) **Abstract**

The invention relates to a computer-implemented method (10) for patient positioning in a medical procedure performed by a medical device (2). According to the method (10), immobilization information (11) is received (S1), wherein the immobilization information (11) is information on immobilization (12) of a patient (3). Then, immobilization (12) of the patient (3) is detected (S2) based on the immobilization information (11) and a skeleton model (13) of the patient (3) is determined (S3). Further, a constraint (14) for patient movement are determined (S4) based on the detected immobilization (12) of the patient (3) and the skeleton model (13) and a constrained optimal patient positioning (15) is determined (S5) based on the skeleton model (13) and the constraint (14) for patient movement. Finally, positioning feedback (17) is provided (S8) based on the constrained optimal patient positioning (15). Further, the invention relates to a corresponding computer-readable medium and a corresponding medical system (1).

## Description

### FIELD OF THE INVENTION

The invention relates to medical procedures, in particular to medical imaging. More particularly, the invention relates to a method for patient positioning in a medical procedure performed by a medical device, to a corresponding computer-readable medium and a medical system.

### BACKGROUND OF THE INVENTION

Patient positioning is crucial to image quality in many domains of medical imaging. State-of-the-art medical imaging systems are often equipped with means to detect inappropriate positioning, propose correction, or an optimum positioning using either the medical image itself (retrospectively) or additional sensors, such as 3D camera (pro-actively). Thus, a camera image, a 3D camera image, a low dose scout image or the imaging modality itself (e.g., in magnetic resonance imaging, or ultrasound imaging) is evaluated typically with a dedicated artificial intelligence or image processing software, as described, e.g., in United States patent application US 2010/0215271 A1.

In recent development, medical imaging equipment has become smarter in a way that it assists technologists in taking a high-quality exam. One crucial part of this assistance is positioning guidance in terms of moving either the patient or the equipment, as described, e.g., in United States patent application US 2021/0118174 A1 or United States patent application US 2021/0244283 A1. Often, adjusting the patient's position is preferred. However, there are situations where this would be impossible, potentially harmful, painful, or at least uncomfortable for the patient, such as when the patient has casts, immobilization devices, or bone dislocation. In these cases, methods proposing an adjustment of the patient position do not help the operator of the medical equipment. Further, a manual interaction, changing the machine's feedback to positioning guidance of the equipment would break the workflow.

### SUMMARY OF THE INVENTION

It is therefore an object of the present invention to provide an improved method for patient positioning, a related computer-readable medium, and a related medical system, in particular providing feasible positioning feedback such as positioning guidance when a patient has an immobilization such as a cast, an immobilization device, or a bone dislocation.

The object of the present invention is solved by the subject matter of the independent claims, wherein further embodiments are incorporated in the dependent claims.

In an aspect of the present invention, a computer-implemented method for patient positioning in a medical procedure performed by a medical device is provided.

In this context, the computer may be a computer of the medical device, a separate computer, or a computer in a cloud. Also, the method may be split on several computers.

According to the method, immobilization information is received. Said immobilization information is information on immobilization of a patient, wherein the patient has to be positioned optimally for the medical procedure. In this context, immobilization may refer to any condition that impedes the patient's ability to move, such as casts, external fixations, internal fixations, or medical conditions such as bone dislocations. Based on said immobilization information, immobilization of the patient is detected.

Further, a skeleton model of the patient is determined. Based on said skeleton model and on the detected immobilization of the patient, a constraint for patient movement is determined. It is understood that it is possible that more than one constraint, in particular a plurality of constraints for patient movement are determined.

Then, a constrained optimal patient positioning is determined based on the skeleton model and the constraint for patient movement. That is, for said constrained optimal patient positioning, the reduced mobility of the patient is taken into account. In other words, the optimal patient positioning is restricted to the movements that are still achievable by the patient.

Finally, positioning feedback is provided based on the constrained optimal patient positioning. Since said positioning feedback is based on the constraint for patient movement, it can actually be applied to the patient, in particular without any further manual interaction to take into account the immobilization of the patient. Compared to positioning feedback that does not take into account the immobilization of the patient, the positioning feedback provided by this method is actually helpful for the medical operator and the positioning advice is improved for patients with immobilizations.

According to an embodiment, the medical procedure is an X-ray imaging procedure, a computed tomography (CT) imaging procedure, a magnetic resonance imaging (MRI) procedure, an ultrasound (US) imaging procedure, an automated breast ultrasound (ABUS) imaging procedure, an image guided therapy (IGT) procedure and/or a radiotherapy procedure. In all of these medical procedures, optimal patient positioning may be necessary to achieve optimal results, and hence the improved positioning feedback for patients with immobilizations improves the results of the medical procedures. For the mentioned medical procedures, the medical devices are an X-ray imaging device, a CT imaging device, an MRI imaging device, a US imaging device, an ABUS imaging device, an IGT device and a radiotherapy device, respectively.

According to an embodiment, the immobilization information comprises a source image. Detecting immobilization of the patient then comprises analyzing the source image. Said source image is a modality image taken by the medical device. In this context, modality image refers to an image taken by the medical device, e.g., an X-ray image, a CT image, or a US image. This modality image may be a preview image or a scout image, in particular taken with a lower radiation dose. Said preview image or scout image may be taken especially for the purpose of determining the optimal patient positioning. In this case, the positioning feedback is provided prior to taking the actual medical image, i.e., in a prospective way. Alternatively, the modality image may be an actual image taken by the medical device. In this case, the positioning feedback may either be an indication that the patient positioning was already good enough, or that a re-take of the medical image with improved patient positioning is proposed, i.e., in a retrospective way. Alternatively, or additionally, the source image is an image from an additional sensor. Said additional sensor may be an optical camera, e.g., an RGB camera or an infrared camera, or a 3D camera, i.e., a depth camera. In this case, the source image may be taken before the actual medical procedure is performed such that prospective guidance can be provided based on the source image. In an example, both a source image from an additional sensor, e.g., a 3D depth camera, and a source image taken by the medical device, e.g., an X-ray image, may be used and analyzed to detect the immobilization of the patient. This may improve the detection of immobilization, in particular since the information provided by the different source images may be complementary: for example, the source image taken by the additional sensor may easily show casts, while the source image from the medical device may easily show internal fixations. In combination, both casts and internal fixations are easily shown, leading to an improved detection of immobilization. The analysis of the source image may be performed, e.g., by image processing techniques, followed by feature recognition, and/or using an artificial intelligence, e.g., a trained neural network.

According to an embodiment, analyzing the source image comprises fitting a default skeleton model to the source image. Said default skeleton model may be an anatomical skeleton model. In particular, the modality image taken by the medical device and/or the image from the additional sensor may be used to fit said default skeleton model to the source image, more particularly by matching internal and/or external features, respectively, to said default skeleton model. Said fitting may be performed, e.g., by image processing techniques, followed by feature recognition, and/or using an artificial intelligence, e.g., a trained neural network. When the skeleton model has been fit to the source image, deviations between the skeleton model and the actual anatomy of the patient may be detected. In particular, bone dislocations may be detected, which may then be analyzed to determine the impact of said bone dislocation on the mobility of the patient and therefore determine an immobilization of the patient due to the bone dislocation. Hence, the overall detection of immobilization is further improved.

According to an embodiment, the immobilization information comprises patient information. Said patient information may be an electronic patient record, previous reports and/or examination requests. For example, said patient information may comprise information on casts, fixations, implants, or medical conditions that interfere with patient mobility. Hence, said patient information may already include information on a plurality of immobilizations of the patient. In order to detect the immobilization of the patient based on the patient information, natural language processing may be used. Alternatively, or additionally, the patient information may comprise mobility parameters which may be directly used to determine immobilization of the patient. The patient information may also be combined with the source images. As an example, the credibility of immobilization detected from a source image may be checked with the information obtained from the patient information. As another example, the patient information may be used to determine whether the source image from the additional sensor is sufficient to determine the immobilization or a source image from the medical device should be obtained to determine the immobilization of the patient: for example, if the patient information comprises information on internal fixations, a source image from the medical device should be obtained, since the source image from the camera does not provide enough information on said internal fixation. Hence, using the patient information, the efficiency and quality of the detection of immobilization is further improved.

According to an embodiment, detecting immobilization of the patient comprises detecting immobilization devices and/or determining immobilized joints, more particularly due to medical conditions. In this context, immobilization devices are devices that constrain the movements of the patient, such as casts, internal fixations and/or external fixations. Immobilized joints are joints that are, at least potentially, immobilized due to medical conditions of the patient, such as fractures, bone dislocations, arthritis, or an aggregation of fluids. When all of said sources of immobilization are taken into account, an accurate assessment of immobilization of the patient is possible.

According to an embodiment, determining the skeleton model of the patient comprises fitting a default skeleton model to the source image. If a default skeleton model is fit to the source image in order to analyze the source image, fitting the default skeleton model to the source image may be performed only once and the results may be used to analyze the source image, to determine the constraint on patient movement, and/or to determine the constrained optimal patient positioning. Said default skeleton model may be an anatomical skeleton model. In particular, the modality image taken by the medical and/or the image from the additional sensor may be used to fit said default skeleton model to the source image, more particularly by matching internal and/or external features, respectively, to the default skeleton model. Said fitting may be performed, e.g., by image processing techniques, followed by feature recognition, and/or using an artificial intelligence, e.g., a trained neural network. Hence, using the source image, an accurate skeleton model can be obtained for the patient.

According to an embodiment, determining the constraint for patient movement comprises spatially correlating detected immobilization with respect to the skeleton model. In other words, the location of the immobilization in relation to the skeleton model is determined. Hence, it can be determined which joints of the skeleton model are affected by said immobilization, which leads to an accurate description of the immobilization of the patient.

According to an embodiment, determining the constrained optimal patient positioning comprises computing an optimum patient position based on the skeleton model and then constraining the optimum patient position based on the constraint for patient movement to obtain the constrained optimal patient positioning. That is, the optimum patient position is first computed without taking into account the immobilization of the patient. This can be done, e.g., by algorithms, by a decision tree, by a random forest, or using an artificial intelligence. In this context, the patient position may be described, e.g., by parameters, wherein the parameters may refer to the flection of a joint and/or to an angle between bones adjacent to a joint. Once said optimum patient position is computed, the movement constraint that has been determined from the detected immobilization of the patient is applied to the optimum patient position. That is, an interference of the immobilization with the proposed optimum patient position is detected. When said movement constraint is applied to the optimum patient position, the constrained optimal patient positioning is obtained. As an example, if the optimum patient position comprises a value for a parameter (e.g., an angle) that is not within the possible movement range of the patient, the value for said parameter that is the closest to the optimum parameter value, but still allowed by the immobilization of the patient, is chosen. Hence, a practicable result is obtained for the patient positioning that takes into account the immobilization. Further, routines for determining the optimum patient position for patients without immobilization may be re-used, allowing for an efficient method for both patients without and with immobilization.

Alternatively, determining the constrained optimal patient positioning comprises determining a constrained set of possible patient positions and determining an optimal choice of the patient position in the constrained set to obtain the constrained optimal patient positioning. That is, the constrained set of possible patient positions is determined first, taking into account the determined immobilization of the patient. Said set of possible patient positions may be given, e.g., as a set of possible patient positioning parameters. Once the set of possible patient positions has been determined, the optimal choice of the patient position is determined in this constrained set. In particular, an algorithm for finding the optimal choice of the patient position may be provided with the constrained set, such that it searches for the optimal patient position only within the allowed range of patient movements. This option yields particularly good results for the case that the parameters of the patient position are correlated with one another.

According to an embodiment, providing positioning feedback comprises providing optimum positioning guidance to achieve the constrained optimal patient positioning. With said guidance, the patient may be positioned accordingly with respect to the medical device and the best possible medical result that is possible given the immobilization of the patient can be obtained.

According to an embodiment, providing optimum positioning guidance comprises proposing patient re-positioning. In other words, a new, optimal patient position is provided. This may be done, e.g., by providing a graphic representation of said optimal patient position, or by providing instructions on how to achieve said optimal patient position, for example to bend joints in a particular way. Performing patient re-positioning is most practicable for the joints that are still mobile, i.e., without or with only little immobilization. Alternatively, or additionally, providing optimum positioning guidance comprises proposing equipment re-positioning. This may also be done by providing a graphic representation of the optimal equipment position with respect to the patient, or by providing instructions on how to move the equipment in order to achieve said optimal equipment position. This is, in particular, most practicable for joints that are immobile, i.e., joints that are affected by the immobilization. Alternatively, or additionally, providing optimum positioning guidance comprises controlling equipment re-positioning. That is, actuators automatically control the equipment to take the optimum equipment position based on the optimal equipment position. Hence, by moving the patient and/or equipment, the constrained optimal patient positioning can be achieved, yielding the best possible medical result.

According to an embodiment, providing positioning feedback comprises providing an assessment on the current patient positioning. As an example, the positioning feedback may comprise a score, e.g., between 0 and 100, for the current patient positioning, wherein a low score refers to a suboptimal positioning and a high score to an optimal positioning, given the immobilization constraint. If said score exceeds a predetermined threshold, no further patient re-positioning will be performed and the medical procedure will be performed. Or, in the case of retrospective evaluation of the immobilization, a decision whether to re-do the medical procedure or not will be based on said score, e.g., based on a comparison with another predetermined threshold. In other words, if the patient is already positioned optimally, or close to optimally, given the immobilization constraint, a re-do of the medical procedure, e.g., a re-take of the medical image, will not be performed, thus saving time, costs, and radiation exposure for the patient.

In another aspect of the present invention, a computer-readable medium is provided. Said computer-readable medium comprises instructions which, when executed by a computer, cause the computer to carry out the method according to the above description. In particular, the instructions correspond to software modules that perform the method according to the above description. Hence, optimal patient positioning, given the immobilization of the patient, is achieved, and therefore optimal results of the medical procedure. Further advantages and details correspond to those given in the above description of the method.

In yet another aspect of the present invention, a medical system is provided. Said medical system comprises a medical device. The medical device may be an X-ray imaging device, a computed tomography (CT) imaging device, a magnetic resonance imaging (MRI) device, an ultrasound (US) imaging device, an automated breast ultrasound (ABUS) imaging device, an image guided therapy (IGT) device, or a radiotherapy device. The medical system further comprises one or more processors configured to carry out the method according to the above description. Hence, optimal patient positioning, given the immobilization of the patient, is achieved, and therefore optimal results of the medical procedure. Further advantages and details correspond to those given in the above description of the method.

It shall be understood that a preferred embodiment of the invention can also be any combination of the dependent claims with the respective independent claim. Further, it has to be noted that embodiments of the invention are described with reference to different subject matters. In particular, some embodiments are described with reference to device type claims whereas other embodiments are described with reference to method type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic view of an embodiment of a medical system;
Fig. 2 shows a flowchart of an embodiment of a method for patient positioning; and
Fig. 3 shows a flowchart of another embodiment of a method for patient positioning.

In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

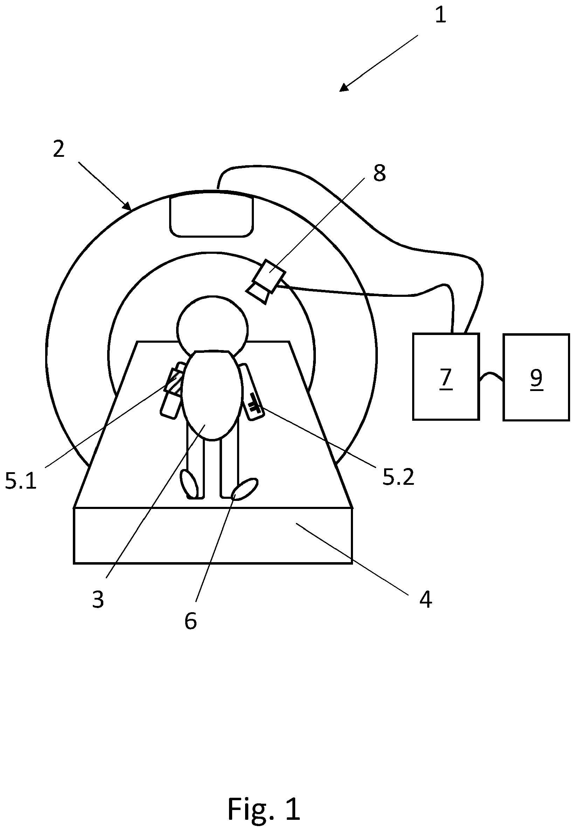

Fig. 1 shows a schematic view of an embodiment of a medical system 1. Said medical system 1 comprises a medical device 2. In the Figure, the medical device 2 is shown as a computed tomography (CT) imaging device, however, the invention also applies to other medical devices such as X-ray imaging devices, magnetic resonance imaging (MRI) devices, ultrasound (US) imaging devices, automated breast ultrasound (ABUS) imaging devices, image guided therapy (IGT) devices, or a radiotherapy devices.

Further, a patient 3 is shown on a patient table 4. The patient 3 is shown with two immobilization devices 5.1 and 5.2, wherein the immobilization device 5.1 is shown as a cast and the immobilization device 5.2 is shown as an internal fixation. Of course, the patient may have other and/or additional immobilization devices. Moreover, the patient 3 is shown to have an immobilized joint 6, e.g., due to a medical condition.

The medical system 1 further comprises a computing unit 7 with one or more processors that is connected to the medical device 2. Also, the medical system 1 comprises an additional sensor 8, which is shown as a camera. The additional sensor 8 may be, e.g., a depth camera and/or an optical camera, such as an RGB camera. The additional sensor 8 is also connected to the computing unit 7 such that the computing unit 7 can receive data from the additional sensor 8.

Moreover, the computing unit 7 is connected to an electronic patient record 9, such that the computing unit 7 can receive data from said electronic patient record 9. The electronic patient record 9 may be part of the medical imaging system 1, but may also be remote from the medical imaging system 1.

The computing unit 7 comprises a computer-readable medium that is configured to cause the computing unit 7 to carry out the below described computer-implemented method for patient positioning.

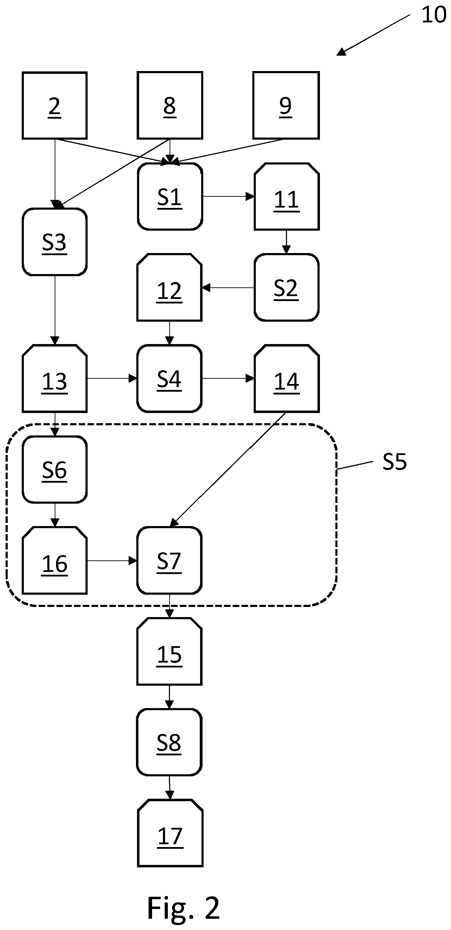

Fig. 2 shows a flowchart of an embodiment of a computer-implemented method 10 for patient positioning.

According to the method 10, immobilization information 11 is received S1, wherein the immobilization information 11 is information on immobilization of the patient 3. In particular, the immobilization information 11 comprises a source image, wherein the source image is a modality image taken by the medical device 2 and/or an image from the additional sensor 8. Alternatively, or additionally, the immobilization information 11 comprises patient information, e.g., from the electronic patient record 9, from previous reports and/or examination requests.

Based on said immobilization information 11, immobilization 12 of the patient 3 is detected S2. Detecting S2 immobilization 12 of the patient 3 may comprise detecting immobilization devices such as casts 5.1, internal fixations 5.2 and/or external fixations, and/or determining immobilized joints 6, more particularly due to medical conditions of the patient 3, such as fractures, bone dislocations, arthritis, or an aggregation of fluids. If the immobilization information 11 comprises source images, detecting S2 immobilization 12 of the patient 3 comprises analyzing said source images. The analysis of the source image may be performed, e.g., by image processing techniques, followed by feature recognition, and/or using an artificial intelligence, e.g., a trained neural network. The analysis of the source image may further comprise fitting a default skeleton model to the source image. Said default skeleton model may be an anatomical skeleton model. In particular, the modality image taken by the medical device 2 and/or the image from the additional sensor 8 may be used to fit said default skeleton model to the source image, more particularly by matching internal and/or external features, respectively, to said default skeleton model. Said fitting may be performed, e.g., by image processing techniques, followed by feature recognition, and/or using an artificial intelligence, e.g., a trained neural network. When the skeleton model has been fit to the source image, deviations between the skeleton model and the actual anatomy of the patient may be detected. In particular, bone dislocations may be detected, which may then be analyzed to determine the impact of said bone dislocation on the mobility of the patient and therefore determine an immobilization 12 of the patient 3 due to the bone dislocation. Hence, the overall detection of immobilization 12 is further improved.

Further, the method 10 comprises determining S3 a skeleton model 13 of the patient. Said skeleton model 13 may be, in particular, determined S3 by fitting a default skeleton model to source images obtained from the medical device 2 and/or the additional camera 8. More particularly, internal and/or external features may be matched to said default skeleton model. The fitting may be performed, e.g., by image processing techniques, followed by feature recognition, and/or using an artificial intelligence, e.g., a trained neural network. When the skeleton model has been fit to the source image, deviations between the skeleton model and the actual anatomy of the patient 3 may be detected. In particular, bone dislocations may be detected, which may then be analyzed to determine the impact of said bone dislocation on the mobility of the patient 3 and therefore determine an immobilization 12 of the patient due to the bone dislocation. Hence, the overall detection of immobilization 12 is further improved.

Based on the detected immobilization 12 and the skeleton model 13, a constraint 14 for patient movement is determined S4. Determining S4 said constraint 14 for patient movement may comprise spatially correlating detected S2 immobilization 12 with respect to the skeleton model 13. In other words, the location of the immobilization 12 in relation to the skeleton model 13 is determined. Hence, it can be determined which joints of the skeleton model 13 are affected by said immobilization 12, which leads to an accurate description of the immobilization 12 of the patient 3.

Then, a constrained optimal patient positioning 15 is determined S5 based on the skeleton model 13 and the constraint 14 for patient movement. In the present embodiment, this comprises computing S6 an optimum patient position 16 based on the skeleton model 13 and then constraining S7 the optimum patient position 16 based on the constraint 14 for patient movement. That is, the optimum patient position 16 is first computed S6 without taking into account the immobilization 12 of the patient 3. This can be done, e.g., by algorithms, by a decision tree, by a random forest, or using an artificial intelligence. In this context, the patient position may be described, e.g., by parameters, wherein the parameters may refer to the flection of a joint and/or to an angle between bones adjacent to a joint. Once said optimum patient position 16 is computed, the movement constraint 14 that has been determined S4 from the detected immobilization 12 of the patient 3 are applied to the optimum patient position 16. That is, an interference of the immobilization 12 with the proposed optimum patient position 16 is detected. When said movement constraint 14 is applied to the optimum patient position 16, the constrained optimal patient positioning 15 is obtained. As an example, if the optimum patient position 16 comprises a value for a parameter (e.g., an angle) that is not within the possible movement range of the patient 3, the value for said parameter that is the closest to the optimum parameter value, but still allowed by the immobilization 12 of the patient 3, is chosen. Hence, a practicable result is obtained for the patient positioning that takes into account the immobilization 12. Further, routines for determining the optimum patient position 16 for patients 3 without immobilization may be re-used, allowing for an efficient method for both patients 3 without and with immobilization 12.

Finally, positioning feedback 17 is provided S8 based on the constrained optimal patient positioning 15. Providing S8 the positioning feedback 17 may comprise providing optimum positioning guidance to achieve the constrained optimal patient positioning 15, including proposing patient re-positioning, proposing equipment re-positioning, and/or controlling equipment re-positioning. By moving the patient and/or equipment, the constrained optimal patient positioning can be achieved, yielding the best possible medical result. Alternatively, or additionally, providing S8 the positioning feedback 17 may comprise providing an assessment on the current patient positioning. Said assessment may be provided as a score for the current patient positioning. If said score exceeds a predetermined threshold, no further patient re-positioning will be performed and the medical procedure will be performed. Or, in the case of retrospective evaluation of the immobilization 12, a decision whether to re-do the medical procedure or not will be based on said score, e.g., based on a comparison with another predetermined threshold. In other words, if the patient is already positioned optimally, or close to optimally, given the immobilization constraint, a re-do of the medical procedure, e.g., a re-take of the medical image, will not be performed, thus saving time, costs, and radiation exposure for the patient.

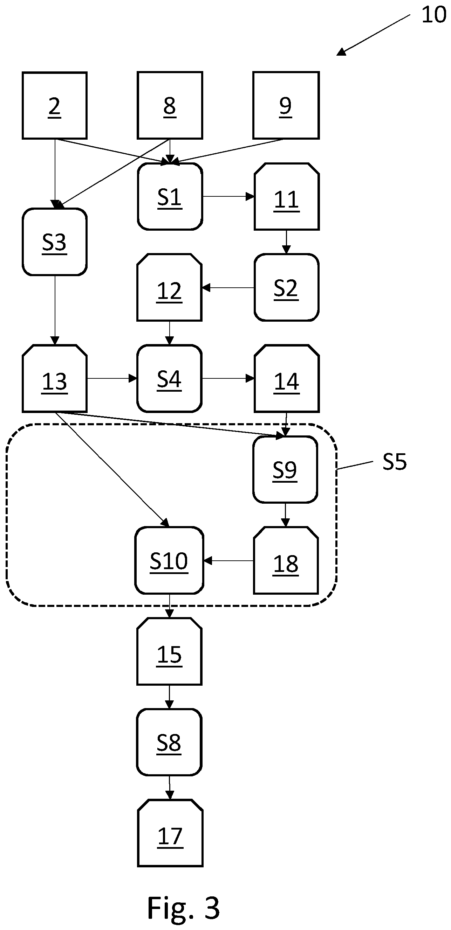

Fig. 3 shows another flowchart of an embodiment of a method 10 for patient positioning.

Compared to the method 10 of Fig. 2, the step of determining S5 the constrained optimal patient positioning 15 is changed. In particular, a constrained set 18 of possible patient positions is determined S9 based on the skeleton model 13 and the constraint 14 for patient movement. Then said constrained set 18 is used, together with the skeleton model 13, to determine S10 an optimal choice of the patient position in the constrained set 18. That is, the constrained set 18 of possible patient positions is determined first, taking into account the determined immobilization 12 of the patient 3. Said set 18 of possible patient positions may be given, e.g., as a set of possible patient positioning parameters. Once the set 18 of possible patient positions has been determined, the optimal choice of the patient position is determined in this constrained set 18. In particular, an algorithm for finding the optimal choice of the patient position may be provided with the constrained set, such that it searches for the optimal patient position only within the allowed range of patient movements. This option yields particularly good results for the case that the parameters of the patient position are correlated with one another.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: medical system
- 2: medical device
- 3: patient
- 4: patient table
- 5: immobilization devices
- 5.1: cast
- 5.2: internal fixation
- 6: immobilized joint
- 7: computing unit
- 8: additional sensor
- 9: electronic patient record
- 10: computer-implemented method
- 11: immobilization information
- 12: immobilization
- 13: skeleton model
- 14: constraint
- 15: constrained optimal patient positioning
- 16: optimum patient position
- 17: positioning feedback
- 18: constrained set

- S 1: receiving immobilization information
- S2: detecting immobilization
- S3: determining a skeleton model
- S4: determining a constraint
- S5: determining constrained optimal patient positioning
- S6: computing an optimum patient position
- S7: constraining the optimum patient position
- S8: providing feedback
- S9: determining the constrained set
- S10: determining an optimal choice

## Claims

1. A computer-implemented method (10) for patient positioning in a medical procedure performed by a medical device (2), comprising:
receiving (S 1) immobilization information (11), wherein the immobilization information (11) is information on immobilization (12) of a patient (3);
detecting (S2) immobilization (12) of the patient (3) based on the immobilization information (11);
determining (S3) a skeleton model (13) of the patient (3);
determining (S4) a constraint (14) for patient movement based on the detected immobilization (12) of the patient (3) and the skeleton model (13);
determining (S5) a constrained optimal patient positioning (15) based on the skeleton model (13) and the constraint (14) for patient movement; and
providing (S8) positioning feedback (17) based on the constrained optimal patient positioning (15).

2. The method (10) according to claim 1, wherein the medical procedure is an X-ray imaging procedure, a computed tomography imaging procedure, a magnetic resonance imaging procedure, an ultrasound imaging procedure, an automated breast ultrasound imaging procedure, an image guided therapy procedure and/or a radiotherapy procedure.

3. The method (10) according to claim 1 or 2, wherein
the immobilization information (11) comprises a source image, the source image being a modality image taken by the medical device (2) and/or an image from an additional sensor (8); and
detecting (S2) immobilization (12) of the patient (3) comprises analyzing the source image.

4. The method (10) according to claim 3, wherein analyzing the source image comprises fitting a default skeleton model to the source image.

5. The method (10) according to any one of claims 1 to 4, wherein the immobilization information (11) comprises patient information like an electronic patient record (9), previous reports and/or examination requests.

6. The method (10) according to any one of claims 1 to 5, wherein detecting (S2) immobilization (12) of the patient (3) comprises detecting immobilization devices (5.1; 5.2) and/or determining immobilized joints (6), more particularly due to medical conditions.

7. The method (10) according to any one of claims 3 to 6, wherein determining (S3) the skeleton model (13) of the patient (3) comprises fitting a default skeleton model to the source image.

8. The method (10) according to any one of claims 1 to 7, wherein determining (S4) the constraint (14) for patient movement comprises spatially correlating detected (S2) immobilization (12) with respect to the skeleton model (13).

9. The method (10) according to any one of claims 1 to 8, wherein determining (S5) the constrained optimal patient positioning (15) comprises:
computing (S6) an optimum patient position (16) based on the skeleton model (13); and
constraining (S7) the optimum patient position (16) based on the constraint (14) for patient movement to obtain the constrained optimal patient positioning (15).

10. The method (10) according to any one of claims 1 to 8, wherein determining (S5) the constrained optimal patient positioning (15) comprises:
determining (S9) a constrained set (18) of possible patient positions; and
determining (S10) an optimal choice of the patient position in the constrained set (18) to obtain the constrained optimal patient positioning (15).

11. The method (10) according to any one of claims 1 to 10, wherein providing (S8) positioning feedback (17) comprises providing optimum positioning guidance to achieve the constrained optimal patient positioning (15).

12. The method (10) according to claim 11, wherein providing optimum positioning guidance comprises proposing patient re-positioning, proposing equipment re-positioning and/or controlling equipment re-positioning.

13. The method (10) according to any one of claims 1 to 12, wherein providing (S8) positioning feedback (17) comprises providing an assessment on the current patient positioning.

14. A computer-readable medium comprising instructions which, when executed by a computer, cause the computer to carry out the method (10) according to any one of claims 1 to 13.

15. A medical system (1), comprising a medical device (2), and one or more processors configured to carry out the method (10) according to any one of claims 1 to 13.
